# EUROPEAN PATENT SPECIFICATION

(11) **EP 3 315 613 B1**
(45) Date of publication and mention of the grant of the patent: **29.07.2020**
(21) Application number: 17198460.2
(22) Date of filing: 26.10.2017
(51) Int. Cl.: C12Q 1/6886

(54) **METHODS AND KITS FOR DIAGNOSING OR ASSESSING THE RISK OF CERVICAL CANCER**
VERFAHREN UND KITS ZUR DIAGNOSE ODER BEURTEILUNG DES RISIKOS VON GEBÄRMUTTERHALSKREBS
PROCÉDÉS ET KITS POUR DIAGNOSTIQUER OU ÉVALUER LE RISQUE DE CANCER DU COL DE L'UTÉRUS

(30) Priority: 26.10.2016 US 201662413088 P
(43) Date of publication of application: 02.05.2018
(73) Proprietor: Chang Gung Memorial Hospital, Linkou, Taoyuan City 33305 (TW)
(72) Inventor: LAI, Chyong-Huey, Taoyuan City 33305 (TW); CHAO, Angel, Taoyuan City 33305 (TW); LIN, Chiao-Yun, Taoyuan City 33305 (TW)
(74) Representative: Cabinet Becker et Associés

(56) References cited:
- CN-A- 102 517 394
- CN-B- 103 361 344
- SHAOSHUAI WANG ET AL: "Association of 42 SNPs with genetic risk for cervical cancer: an extensive meta-analysis", BMC MEDICAL GENETICS, BIOMED CENTRAL, LONDON, GB, vol. 16, no. 1, 15 April 2015 (2015-04-15) , page 25, XP021217000, ISSN: 1471-2350, DOI: 10.1186/S12881-015-0168-Z
- Anonymous: "Axiom (TM) Genome-Wide CHB 1 Array Plate", , 1 January 2011 (2011-01-01), XP055451048, Retrieved from the Internet: URL:https://tools.thermofisher.com/content /sfs/manuals/axiom_chb_arrayplate_insert.p df [retrieved on 2018-02-14]
- Anonymous: "Reference SNP (refSNP) Cluster Report: rs17024091", NCBI, 28 August 2004 (2004-08-28), XP055451290, Retrieved from the Internet: URL:https://www.ncbi.nlm.nih.gov/projects/ SNP/snp_ref.cgi?searchType=adhoc_search&ty pe=rs&rs=rs17024091 [retrieved on 2018-02-14]
- anonymous: "Reference SNP (refSNP) Cluster Report: rs2046542", NCBI, 19 April 2001 (2001-04-19), XP055479293, Retrieved from the Internet: URL:https://www.ncbi.nlm.nih.gov/SNP/snp_r ef.cgi?rs=2046542&pt=1PqYSE1PNE4gI4-Qzyc-h 1tUwYQvW4OVUJS78Z_AnvgYnok5j [retrieved on 2018-05-29]
- Anonymous: "Reference SNP (refSNP) Cluster Report: rs1386286", NCBI, 1 November 2000 (2000-11-01), XP055479297, Retrieved from the Internet: URL:https://www.ncbi.nlm.nih.gov/SNP/snp_r ef.cgi?rs=1386286&pt=1piy_E3zS5nS8rtkXedbe jTn1PGKniykAVI5SRWeCEW5XyZk4 [retrieved on 2018-05-29]

## Description

### BACKGROUND OF THE INVENTION

Human papillomavirus (HPV) testing is widely used for cervical cancer screening. Women with vaginal HPV infection and normal cervical cytology are more likely to develop cervical cancer and/or cervical intraepithelial neoplasia (CIN) compared to women without vaginal HIV infection and normal cervical cytology. The diagnosis of HPV infection causes substantial anxiety in women as the risk of cervical cancer increases. However, apart from the viral infection, there are host risk factors that are associated with cervical cancer development.

S Wang *et al.* identified 42 single nucleotide polymorphisms (SNPs) associated with cervical cancer using three genetic models: the allele model, dominant model and recessive model, in which rs 1048943 showed highly significant association with cervical cancer in all three models. CN 102517394 A discloses a kit for diagnosing cervical cancer by detecting four mononucleotide polymorphisms: Arg72Pro(rs1042522) on gene P53, deletion of gene GSTM1 (null/present), deletion of gene GSTT1 gene (null/present) and C677T(rs1801133) on gene MTHFR. CN 103361344B shows rsl3117307, rs4282438, rs9277952, rs2116260, rs3129275, rs3117008, rs9391756, rs213210, rs4282438, rs9277952, rs8067378 are associated with cervical cancer.The identification of the host risk factor is important for assessing the risk of developing cervical cancer, as well as alleviating the anxiety in women with vaginal HPV infection. There is an unmet need for an economical and accurate laboratory diagnostic test for predicting the risk of cervical cancer and/or cervical intraepithelial neoplasia (CIN) development and the present invention satisfy this and other needs.

### BRIEF SUMMARY OF THE INVENTION

In one embodiment, the present invention discloses methods for assessing the risk of developing cervical cancer or CIN in a subject, comprising the steps of detecting at least one single nucleotide polymorphism (SNP) selected from rs2046542 (SEQ ID NO:2) or rs1386286 (SEQ ID NO:3), in the sample of the subject, wherein the presence of at least one SNP is indicative of an increased risk for developing cervical cancer or CIN in the subject.

The present description further discloses methods for assessing the risk of developing cervical cancer or CIN in a subject, comprising the steps of detecting at least two SNPs selected from rs8067378 (SEQ ID NO:10), rs4282438 (SEQ ID NO:11), or rs1386286 (SEQ ID NO:3) in the sample of the subject, wherein the presence of at least two SNPs is indicative of an increased risk for developing cervical cancer or CIN in the subject.

It is also described kits for assessing the risk of developing cervical cancer or CIN in a subject, comprising an agent for detecting at least one SNP selected from rs17024091, rs2046542, rsl386286, rs3097662, rs3117221, rs7759943, rsl333934, rsl3347411 or rs11651242 in the sample of a subject.

The present disclosure further provides kits for assessing the risk of developing cervical cancer in a subject, comprising an agent for detecting at least two of the following SNPs: rs8067378, rs4282438, or rsl386286 in the sample of a subject.

In one embodiment, the kit described herein further comprises a label indicates that the agent is for detecting at least one SNP for assessing the risk of cervical cancer/CIN development.

The present description also provides a set of SNPs for assessing the risk of developing cervical cancer/CIN, wherein the SNPs are selected from the group consisting of rs2046542, rsl386286, the combination of rs8067378 and rs4282438, the combination of rs8067378 and rsl386286 , the combination of rs4282438 and rsl386286, and the combination of rs8067378, rs4282438, and rsl386286. Methods for assessing the risk of developing cervical cancer in a subject, comprising the step of detecting at least one SNP from the SNP set described above are provided. Also provided is a microarray for assessing the risk of developing cervical cancer or CIN, wherein the microarray comprises a set of polynucleotide to hybridize with the polynucleotide of at least one of the following SNP: rs2046542, rsl386286, the combination of rs8067378 and rs4282438, the combination of rs8067378 and rsl386286 , the combination of rs4282438 and rs1386286, or the combination of rs8067378, rs4282438, and rs1386286.

The invention will become more apparent when read with the accompanying figures and detailed description which follow.

### BRIEF DESCRIPTION OF THE DRAWINGS

Illustrative embodiments of the present invention are described in detail below with reference to the following Figures:
FIG. 1A to FIG. 1C are flow charts illustrating the study design of the discovery cohort, verification cohort and prediction cohort of the Genome-Wide Association Study (GWAS) in Example 1.
Fig. 2 is a Manhattan plot, illustrating the SNPs associated with cervical intraepithelial neoplasia grade 2 or higher (CIN2⁺) based on Genome-Wide association study of Example 1.
Fig. 3 is a graph showing the cumulative incidence of cervical cancer progression in patients with no or one risk SNP (≤1 SNPs) and patients with more than one SNP (>1 SNPs).

### DETAILED DESCRIPTION OF THE INVENTION

As used herein, the articles "a" and "an" refer to one or more than one (*i.e*., at least one) of the grammatical object of the article.

The term "subject" may refer to a vertebrate suspected of having cervical cancer. Subjects include warm-blooded animals, such as mammals, such as a primate, and, more preferably, a human. Non-human primates are subjects as well. The term subject includes domesticated animals, such as cats, dogs, etc., livestock (for example, cattle, horses, pigs, sheep, goats, etc.) and laboratory animals (for example, mouse, rabbit, rat, gerbil, guinea pig, etc.). In an embodiment, the subject has vaginal Human papillomavirus (HPV) infection.

All numbers herein may be understood as modified by "about." As used herein, the term "about," when referring to a measurable value, is meant to encompass variations of ± 10%, preferably ± 5%, more preferably ± 1%, and even more preferably ± 0.1% from the specified value, as such variations are appropriate to percentage of homology unless otherwise specified.

A multiple single nucleotide polymorphisms (SNPs) for assessing the risk of developing cervical cancer/CIN as described herein includes at least one polynucleotide sequences of SEQ ID NOS: 1 to 11.

**Table 1**

| Gene Bank accession of SNP in NCBI | Polynucleotide Containing SNP |
|---|---|
| rs17024091 | SEQ ID NO: 1 |
| rs2046542 | SEQ ID NO:2 |
| rs1386286 | SEQ ID NO:3 |
| rs3097662 | SEQ ID NO:4 |
| rs3117221 | SEQ ID NO:5 |
| rs7759943 | SEQ ID NO:6 |
| rs1333934 | SEQ ID NO:7 |
| rs13347411 | SEQ ID NO:8 |
| rs11651242 | SEQ ID NO:9 |
| rs8067378 | SEQ ID NO:10 |
| rs4282438 | (SEQ ID NO:11) |

The GenBank accession No. of an SNP in the National Center for Biotechnology Information (NCBI) database indicates a sequence and a position of the SNP. Those skilled in the art may easily identify the sequence and the position of the SNP using the GenBank accession No. The specific sequences corresponding to the rs No. of the SNP registered in NCBI may change over time. It is obvious to those skilled in the art that the sequences are within the scope of the present invention, even if the corresponding rs number changes. The nucleotide sequences of SEQ ID NOS: 1 to 11 are polymorphic sequences. A polymorphic sequence is a polynucleotide sequence including a polymorphic site representing SNP. The polynucleotide sequences can be DNA or RNA.

The risk alleles are described in Table 2.

### METHODS FOR ASSESSING THE RISK OF DEVELOPING CERVICAL CANCER/CIN

The diagnosing method includes isolating DNA from a sample of a subject, determining a base sequence at a polymorphic site of the DNA, and judging that the subject has cervical cancer/CIN or has a high incidence/probability of cervical cancer/CIN when the base sequence includes at least one SNP selected from SEQ ID NO:1 to SEQ ID NO:9.

In certain embodiments, methods for assessing the risk of developing cervical cancer or CIN in a subject are provided, comprising the steps of detecting at least two SNPs selected from rs8067378, rs4282438 or rsl386286 in the sample of the subject, wherein the presence of at least two SNPs is indicative of having an increased risk for developing cervical cancer or CIN in the subject. In an exemplary embodiment, the method comprises the step of detecting a first SNP at rs8067378 and a second SNP at rs4282438. In another embodiment, the method comprises the step of detecting a first SNP at rs8067378 and a second SNP at rsl386286. In yet another embodiment, the method comprises the step of detecting a first SNP at rs4282438 and a second SNP at rsl386286. In yet another embodiment, the method comprises the step of detecting a first SNP at rs4282438, a second SNP at rsl386286 and a third SNP at rs8067378.

The present invention is based, in part, on the identification of particular SNP in a test sample. Some embodiments of the present invention are directed to methods of assessing whether a subject has, or is at risk for developing, cervical cancer, comprising the identification of particular SNP in a test sample. Non limiting examples of the sample include DNA from peripheral white blood cells, serum, cell, tissue, or biopsy.

In some embodiments, molecular profiling can also include identifying a genetic variant, such as a mutation, polymorphism (such as a SNP), deletion, or insertion of a target. For example, identifying a SNP in a gene can be determined by microarray analysis, real-time PCR, or sequencing. Other methods disclosed herein can also be used to identify variants of one or more targets.

The polymorphisms can be detected by any available method, including amplification, hybridization to a probe or array, or the like, see B Sobrino et al, SNPs in forensic genetics: a review on SNP typing methodologies, Forensic Science International, Volume 154, Issues 2-3, 25 November 2005, Pages 181-194. In one specific embodiment, SNP detection includes amplifying the polymorphism, linked locus or a sequence associated therewith (e.g., flanking sequences, transcribed sequences or the like) and detecting the resulting amplicon. For example, in one embodiment, amplifying includes a) admixing an amplification primer or amplification primer pair with a nucleic acid template isolated from the organism or biological sample. The primer or primer pair can be complementary or partially complementary to a region proximal to or including the polymorphism or linked locus, and are capable of initiating nucleic acid polymerization by a polymerase on the nucleic acid template. The primer or primer pair is extended in a DNA polymerization reaction comprising a polymerase and the template nucleic acid to generate the amplicon. In certain aspects, the amplicon is optionally detected by a process that includes hybridizing the amplicon to an array, digesting the amplicon with a restriction enzyme, or real-time PCR analysis. Optionally, the amplicon can be fully or partially sequenced, e.g., by hybridization. Typically, amplification can include performing a polymerase chain reaction (PCR), reverse transcriptase PCR (RT-PCR), or ligase chain reaction (LCR) using nucleic acid isolated from the organism or biological sample as a template in the PCR, RT-PCR, or LCR. Other technologies can be substituted for amplification, e.g., use of branched DNA (bDNA) probes.

These SNPs include SNPs associated with, e.g., the following genes: TMEM178: transmembrane protein 178A; RPL32P9: ribosomal protein L32 pseudogene 9; RING1: ring finger protein 1; VPS52: vacuolar protein sorting 52; NPAP1P4: nuclear pore associated protein 1 pseudogene 4; MTND2P9: mitochondrially encoded NADH: ubiquinone oxidoreductase core subunit 2 pseudogene 9; TNFRSF13B: tumor necrosis factor receptor superfamily, member 13B; IKZF3: IKAROS family zinc finger 3; ZPBP2: zona pellucida binding protein 2; GSDMB: gasdermin B; ORMDL3: ORMl-like 3; LRRC3C: leucine rich repeat containing 3C. Polymorphisms linked to these genes are, accordingly, also preferred SNPs that can be associated with cervical cancer polymorphisms.

### KITS FOR ASSESSING THE RISK OF DEVELOPING OF CERVICAL CANCER AND/OR CIN

The present description also provides kits for use in predicting the risk of developing cervical cancer and/or CIN. The kit comprises an agent for detecting at least one of the following SNPs located at rsl7024091, rs2046542, rs1386286, rs3097662, rs3117221, rs7759943, rs1333934, rs13347411 or rs11651242.

The present description further provides kits for assessing the risk of developing cervical cancer in a subject, comprising an agent for detecting at least two of the following SNPs: rs8067378, rs4282438, and rsl386286. In an exemplary embodiment, the kit comprises a first agent for detecting a first SNP at rs8067378 and a second agent for detecting a second SNP at rs4282438. In another embodiment, the kit comprises a first agent for detecting a first SNP at rs8067378 and a second agent for detecting a second SNP at rsl386286. In yet another embodiment, the kit comprises a first agent for detecting a first SNP at rs4282438 and a second agent for detecting a second SNP at rsl386286. In yet another embodiment, the kit comprises a first agent for detecting a first SNP at rs4282438, a second agent for detecting a second SNP at rsl386286 and a third agent for detecting a third SNP at rs8067378.

In one embodiment, the kit further comprises an instruction for using the agent to assess the risk of developing cervical cancer or CIN. The agent can be an agent known in the art for detecting SNP.

Non-limiting examples of the agent include a primer set for isolating and amplifying DNA or a hybridization probe capable of detecting at least one SNP. The term "primer" refers to an oligonucleotide used in a polymerase chain reaction (PCR) reaction. The appropriate primer set may be easily designed by those skilled in the art with reference to the sequences described herein.

In some embodiments, use of a microarray is desirable. A microarray is a microscopic, ordered array of nucleic acids, proteins, small molecules, cells or other substances that enables parallel analysis of complex biochemical samples. Microarrays can be fabricated using a variety of technologies, including printing with fine-pointed pins onto glass slides, photolithography using pre-made masks, photolithography using dynamic micromirror devices, ink-jet printing, or electrochemistry on microelectrode arrays.

A microarray for cervical cancer diagnosis according to certain embodiments of the present disclosure includes allele-specific oligonucleotide (ASO) probes (i.e., polynucleotide hybridized with the polynucleotide of SEQ ID NOS:1-11). The ASO probes may be immobilized on a substrate coated with an active group selected among amino-silane, poly-L-lysine and aldehyde. Also, the substrate may be composed of a silicon wafer, glass, quartz, metal or plastic. The method of immobilizing the polynucleotide on the substrate may be either micropipetting using piezoelectric or a method using a pin-shaped spotter

A kit for assessing the risk of developing cervical cancer according to an embodiment of the present disclosure includes the microarray described herein.

Embodiments of the present invention are illustrated by the following examples, which are not to be construed in any way as imposing limitations upon the scope thereof. During the studies described in the following examples, conventional procedures were followed, unless otherwise stated. Some of the procedures are described below for illustrative purpose.

### Example 1: Genome-Wide Association Study (GWAS)

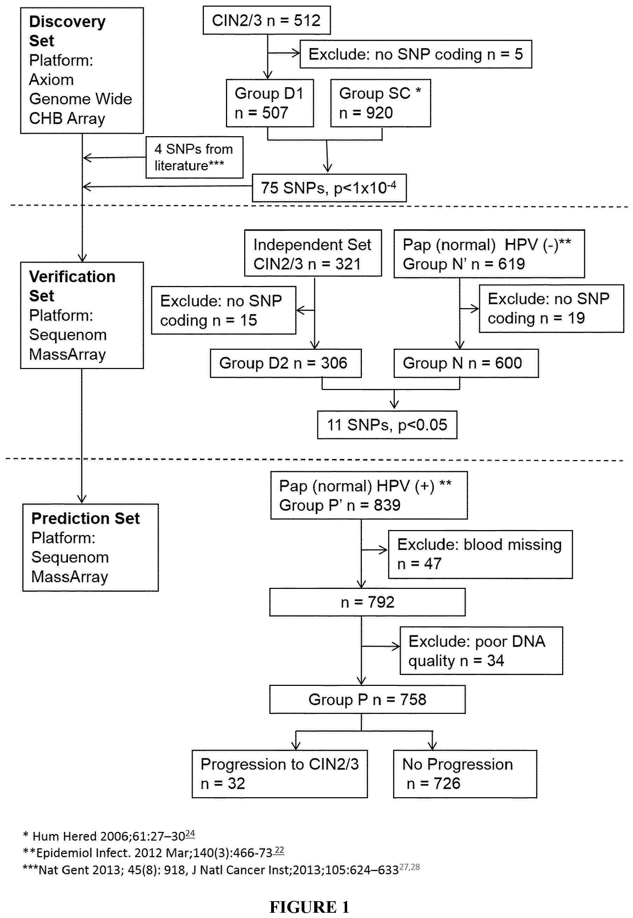

As illustrated in FIG. 1A, a genome-wide association study (GWAS) of 642,832 single nucleotide polymorphisms (SNPs) by Affymetrix Axiom™ Genome-Wide Human Arrays was conducted on 507 cases with histological diagnosis of CIN2⁺ (group D1) versus 920 female controls (group C) as a discovery set.

The identified 75 CIN2⁺-associated SNPs from GWAS and 4 cervical cancer related SNPs from the literature were verified in an independent verification cohort as shown in Fig. 1B. Group N [n = 600] were HPV-negative/ normal cytology women from a population-based cervical cytology and HPV co-test study whereas group D2 [n = 306] are women with CIN2⁺.

In the prediction cohort (FIG. 1C), subjects with HPV-positive/normal (group P, n = 758) were followed up and served as the prediction cohort. The predictive validity was analyzed by logistic regression and receiver operating characteristic (ROC) curve analysis.

### Results

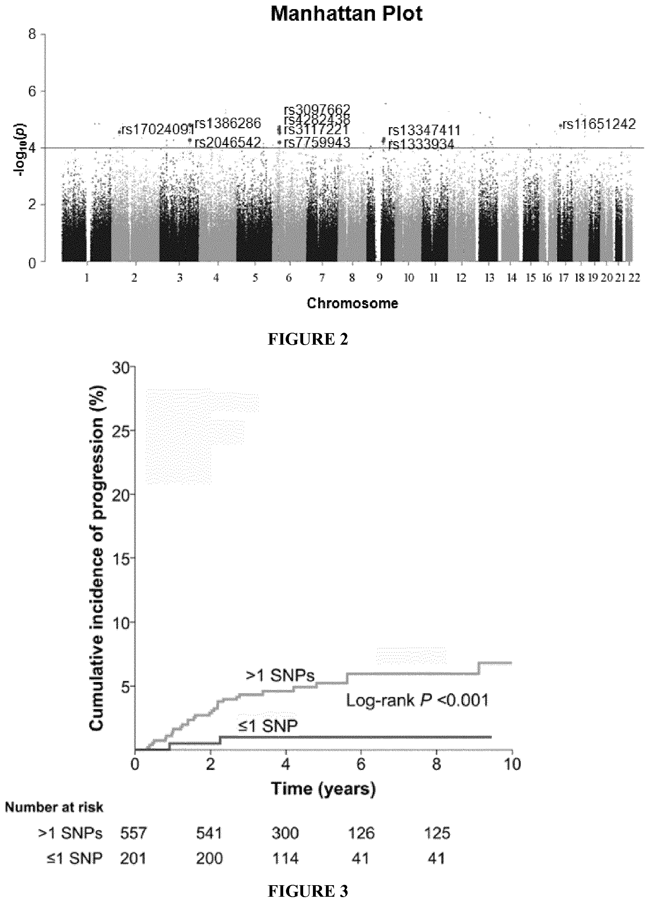

Thirty-two individuals of the group P progressed to CIN2+ (median follow-up: 56.5 months, range 30.7- 119.4). Ten out of seventy-five SNPs with trend test p values < 10⁻⁴ identified from the discovery cohort along with rs8067378 reported from literature were validated in the verification set and approved to be associated with progression to CIN2⁺ (Figure 2 and Table 2).

**Table 2. SNPs identified from discovery cohort and validated in the verification cohort that are associated with the risk of developing CIN ²⁺ or cervical cancer.**

| dbSNP rsID | Chromosome | Physical Position (bp) | Major/ Minor Allele | Minor Allele Frequency in Case (N = 507) | Minor Allele Frequency in Control (N = 920) | Risk Allele | Trend Test P-value | Nearby Gene |
|---|---|---|---|---|---|---|---|---|
| rs17024091 | 2 | 39847169 | T/C | 0.1578 | 0.2241 | T | 3.67x10⁻⁵ | *TMEM178* |
| rs2046542 | 3 | 149832485 | C/T | 0.2875 | 0.3617 | C | 6.92x10⁻⁵ *L* | *LOC730021, RPL32P9* |
| rs1386286 | 3 | 149843853 | G/A | 0.2747 | 0.3513 | G | 2.24x10⁻⁵ *L* | *LOC730021, RPL32P9* |
| rs3097662 | 6 | 33020777 | T/C | 0.1907 | 0.1317 | C | 2.71x10⁻⁵ | *HLA-DPA1,HLA-DPB1,HLA-DPB2* |
| rs3117221 | 6 | 33061947 | T/C | 0.4772 | 0.3967 | C | 3.42x10⁻⁵ | *HLA-DPA1,HLA-DPB1,HLA-DPB2* |
| rs4282438 | 6 | 33072172 | T/G | 0.3901 | 0.4707 | T | 3.23x10⁻⁵ | *HLA-DPA1,HLA-DPB1,HLA-DPB2* |
| rs7759943 | 6 | 33195154 | G/A | 0.1028 | 0.062 | A | 9.92x10⁻⁵ | *RING1, VPS52* |
| | | | | | | | | |
| rs1333934 | 9 | 82935097 | A/C | 0.4448 | 0.37 | C | 7.58x10⁻⁵ | *NPAPIP4* |
| | | | | | | | | |
| rs13347411 | 9 | 83205419 | A/G | 0.3587 | 0.4354 | A | 7.32x10⁻⁵ | *MTND2P9* |
| rs11651242 | 17 | 16854546 | T/G | 0.1755 | 0.2448 | T | 2.14x10⁻⁵ | *TNFRSF13B* |
| rs8067378* | 17 | 38051348 | A/G | 0.2931 | 0.263 | G | 0.0873 | *IKZF3, ZPBP2, GSDMB, ORMDL3, LRRC3C* |

A risk-predictive SNP panel of rs8067378, rs4282438, and rs1386286 was generated from the discovery and verification set combining HPV types and tested in group P (sensitivity 0.844, negative predictive value 0.986, ROC 0.725) (Table 3).

**Table 3. Distribution of risk-alleles and observed progression to CIN2⁺ in group P stratified by HPV types**

| **Risk SNP(s)** | **HPV high-risk^{a}** | | **HPV low-riskb** | |
|---|---|---|---|---|
| | **N** | **No. of Progression (%)** | **N** | **No. of Progression (%)** |
| **0 SNP** | 6 | 0 (0) | 5 | 0 (0) |
| **1 SNP** | | | | |
| **(a1) rs8067378, 1 risk allele** | 4 | 0 (0) | 3 | 0 (0) |
| **(a2) rs8067378, 2 risk alleles** | 0 | 0 (0) | 1 | 0 (0) |
| **(b1) rs4282438, 1 risk allele** | 19 | 0 (0) | 15 | 0 (0) |
| **(b2) rs4282438, 2 risk alleles** | 6 | 0 (0) | 7 | 0 (0) |
| **(c1) rs1386286, 1 risk allele** | 38 | 1 (2.6) | 27 | 0 (0) |
| **(c2) rs1386286, 2 risk alleles** | 24 | 1 (4.2) | 11 | 0 (0) |
| **2 SNPs** | | | | |
| **(a1)+(b1)** | 13 | 1 (7.7) | 7 | 0 (0) |
| **(a1)+(b2)** | 5 | 0 (0) | 4 | 0 (0) |
| **(a1)+(c1)** | 23 | 0 (0) | 9 | 0 (0) |
| **(a1)+(c2)** | 20 | 1 (5.0) | 13 | 0 (0) |
| **(a2)+(b1)** | 0 | 0 | 1 | 0 (0) |
| **(a2)+(b2)** | 2 | 0 (0) | 0 | 0 (0) |
| **(a2)+(c1)** | 2 | 0 (0) | 0 | 0 (0) |
| **(a2)+(c2)** | 1 | 0 (0) | 2 | 0 (0) |
| **(b1)+(c1)** | 43 | 3 (7.0) | 34 | 0 (0) |
| **(b1)+(c2)** | 48 | 5 (10.4) | 46 | 3 (6.5) |
| **(b2)+(c1)** | 27 | 3 (11.1) | 22 | 0 (0) |
| **(b2)+(c2)** | 31 | 3 (9.7) | 30 | 0 (0) |
| **3 SNPs** | | | | |
| **(a1)+(b1)+(c1)** | 35 | 3 (8.6) | 29 | 0 (0) |
| **(a1)+(b1)+(c2)** | 29 | 3 (10.3) | 24 | 1 (4.2) |
| **(a1)+(b2)+(c1)** | 20 | 2 (10.0) | 19 | 0 (0) |
| **(a1)+(b2)+(c2)** | 15 | 2 (13.3) | 11 | 0 (0) |
| **(a2)+(b1)+(c1)** | 2 | 0 (0) | 0 | 0 (0) |
| **(a2)+(b1)+(c2)** | 6 | 0 (0) | 3 | 0 (0) |
| **(a2)+(b2)+(c1)** | 5 | 0 (0) | 3 | 0 (0) |
| **(a2)+(b2)+(c2)** | 5 | 0 (0) | 3 | 0 (0) |

| | | | | |
|---|---|---|---|---|
| ^{a}HPV16,18, 31,33,35,39,45, 51, 52, 56, 58, 59, 66, and 68. ^{b}HPV6, 11, 26, 32, 37, 42, 43, 44, 53, 54, 55, 61, 62, 67, 69, 70, 71, 72, 74, 81, 82, 83, 84, L1AE5 | | | | |

As illustrated in Fig. 3, women carried ≤ 1 risk-allele had significantly lower hazard ratio (0.21 [0.05-0.86], p = 0.031) of cervical cancer progression than those with >1 risk-allele (1.7% for ≤ 1 risk-allele vs. 10.7% for >1 risk-allele).

## Claims

1. A method for assessing the risk of developing cervical cancer in a subject, comprising the step of detecting at least one single nucleotide polymorphism (SNP) marker selected from rs2046542, rs1386286, a combination of rs8067378 and rs1386286, a combination of rs4282438 and rs1386286, or a combination of rs8067378, rs4282438, and rs1386286 in the subject, wherein the presence of a risk allele as listed in Table 2 in at least one of the SNP markers is indicative of an increased risk for developing cervical cancer in the subject.

2. The method of claim 1, wherein the SNP marker is detected in a serum sample.

3. The method of claim 1 or 2, wherein the subject has vaginal human papilloma virus infection.

## Patentansprüche

1. Ein Verfahren zur Beurteilung des Risikos einer Entwicklung von Gebärmutterhalskrebs in einer Person, umfassend den Schritt des Nachweises wenigstens eines Einzelnukleotid-Polymorphismus-(SNP)-Markers, ausgewählt aus rs2046542, rs1386286, einer Kombination aus rs8067378 und rs1386286, einer Kombination aus rs428238 und rs1386286 oder einer Kombination aus rs8067378, rs4282438 und rs1386286, in der Person, wobei das Vorhandensein eines Risikoallels, wie in Tabelle 2 angeführt, in wenigstens einem der SNP-Marker ein erhöhtes Risiko einer Entwicklung von Gebärmutterhalskrebs in der Person anzeigt.

2. Das Verfahren nach Anspruch 1, wobei der SNP-Marker in einer Serumprobe nachgewiesen wird.

3. Das Verfahren nach Anspruch 1 oder 2, wobei die Person eine Vaginalinfektion mit humanem Papillomavirus hat.

## Revendications

1. Méthode pour déterminer le risque de développer un cancer du col de l'utérus chez un sujet, comprenant l'étape de détection d'au moins un marqueur de polymorphisme mononucléotidique (SNP) choisi parmi rs2046542, rs1386286, une combinaison de rs8067378 et rs1386286, une combinaison de rs4282438 et rs1386286, ou une combinaison de rs8067378, rs4282438 et rs1386286 chez le sujet, dans laquelle la présence d'un allèle de risque tel que listé dans le Tableau 2 dans au moins l'un des marqueurs SNP est indicative d'un risque accru de développer un cancer du col de l'utérus chez le sujet.

2. Méthode selon la revendication 1, dans laquelle le marqueur SNP est détecté dans un échantillon de sérum.

3. Méthode selon la revendication 1 ou 2, dans laquelle le sujet a une infection vaginale par le virus du papillome humain.


### SEQUENCE LISTING

<110> Chang Gung Memorial Hospital, Linkou
<120> METHODS AND KITS FOR DIAGNOSING OR ASSESSING THE RISK OF CERVICAL CANCER
<130> B2608EP
<160> 11
<170> PatentIn version 3.5
<210> 1
   <211> 51
   <212> DNA
   <213> Homo sapiens
<220>
   <221> misc_feature
   <222> (26)..(26)
   <223> SNP rs17024091; n is c or t
<400> 1
   tatgagaaaa tacatacatg gtattnagca ccaatacttg atttcctata t 51
<210> 2
   <211> 51
   <212> DNA
   <213> Homo sapiens
<220>
   <221> misc_feature
   <222> (26)..(26)
   <223> SNP rs2046542; n is a or g
<400> 2
   aaaaaagata gtgggttggt cagtgntttc ttacggctgc tctcacagat t 51
<210> 3
   <211> 51
   <212> DNA
   <213> Homo sapiens
<220>
   <221> misc_feature
   <222> (26)..(26)
   <223> SNP rs1386286; n is a or g
<400> 3
   aaaaatgtga gcaggatggg aaaatncggc aaatgatggt cacatattct c 51
<210> 4
   <211> 51
   <212> DNA
   <213> Homo sapiens
<220>
   <221> misc_feature
   <222> (26).. (26)
   <223> SNP rs3097662; n is c or t
<400> 4
   ctagatcaca tagacaccca ttattncatc cagttgttta ttttccccac c 51
<210> 5
   <211> 51
   <212> DNA
   <213> Homo sapiens
<220>
   <221> misc_feature
   <222> (26)..(26)
   <223> SNP rs3117221; n is a or g
<400> 5
   aatcctctag accctaaact cagtgnggga ccctcaccat ccgtgtgcgg c 51
<210> 6
   <211> 51
   <212> DNA
   <213> Homo sapiens
<220>
   <221> misc_feature
   <222> (26)..(26)
   <223> SNP rs7759943; n is a or g
<400> 6
   ttgttcttat aatgggatac tatacngcag ttaaatgaat tatagacata t 51
<210> 7
   <211> 51
   <212> DNA
   <213> Homo sapiens
<220>
   <221> misc_feature
   <222> (26)..(26)
   <223> SNP rs1333934; n is g or t
<400> 7
   ttcaatcagg aaactttctg tagganattg accttggtct gtaaaggaaa g 51
<210> 8
   <211> 51
   <212> DNA
   <213> Homo sapiens
<220>
   <221> misc_feature
   <222> (26).. (26)
   <223> SNP rs13347411; n is a or g
<400> 8
   tatattgtta ctttataaca gtttcngatg cagcaaggca atattatctc t 51
<210> 9
   <211> 51
   <212> DNA
   <213> Homo sapiens
<220>
   <221> misc_feature
   <222> (26)..(26)
   <223> SNP rs11651242; n is a, g or t
<400> 9
   gtcactcagc ctctctgagt tccccncaat aagtattcat gaaggactta c 51
<210> 10
   <211> 51
   <212> DNA
   <213> Homo sapiens
<220>
   <221> misc_feature
   <222> (26)..(26)
   <223> SNP rs8067378; n is a or g
<400> 10
   aaaaaatatt tgtaacgtta taaatnggga aaaacgttta tatcactgcc a 51
<210> 11
   <211> 51
   <212> DNA
   <213> Homo sapiens
<220>
   <221> misc_feature
   <222> (26)..(26)
   <223> SNP rs4282438; n is g or t
<400> 11
   acaggttaag tatccatact ccaagnaaat ttggagagca tttcccagag a 51